(19)

Europäisches
Patentamt

European
Patent Office

Office européen
des brevets

(11) **EP 2 766 729 B1**

(12) **EUROPEAN PATENT SPECIFICATION**

(45) Date of publication and mention
of the grant of the patent:
**05.12.2018 Bulletin 2018/49**

(51) Int Cl.:
***G01N 33/564*** (2006.01)     ***G01N 33/574*** (2006.01)

(21) Application number: **12780857.4**

(22) Date of filing: **11.10.2012**

(86) International application number:
**PCT/IB2012/055509**

(87) International publication number:
**WO 2013/054281 (18.04.2013 Gazette 2013/16)**

(54) **METHOD TO DIAGNOSE THE PRESENCE OF PROSTATE CANCER**

VERFAHREN ZUR DIAGNOSTIZIERUNG VON PROSTATAKREBS

PROCÉDÉ DE DIAGNOSTIC DE LA PRÉSENCE D'UN CANCER DE LA PROSTATE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.10.2011 IT PD20110323**

(43) Date of publication of application:
**20.08.2014 Bulletin 2014/34**

(73) Proprietor: **Xeptagen SPA
30175 Marghera (VE) (IT)**

(72) Inventors:
- **FASSINA, Giorgio
  30175 Marghera (VE) (IT)**
- **GALLOTTA, Andrea
  30175 Marghera (VE) (IT)**

(74) Representative: **Vinci, Marcello
Ufficio Veneto Brevetti
Via Sorio 116
35141 Padova (IT)**

(56) References cited:
- **Michela Verna ET AL: "Improved Detetction Of
  Low-Grade Prostate Cancer By PSA-IgM
  Assessment", Advanced Cancer Research 30;
  Abstract 196, 17 June 2010 (2010-06-17),
  XP055017149, Retrieved from the Internet:
  URL:http://ar.iiarjournals.org/content/30/
  4/1375.full.pdf+html [retrieved on 2012-01-23]**

- **BENEDUCE ET AL: "Detection of
  prostate-specific antigen coupled to
  immunoglobulin M in prostate cancer patients",
  CANCER DETECTION AND PREVENTION,
  ELSEVIER SCIENCE, vol. 31, no. 5, 26 November
  2007 (2007-11-26), pages 402-407, XP022370706,
  ISSN: 0361-090X, DOI:
  10.1016/J.CDP.2007.10.005**
- **Xeptagen: "Prostate-IC", , 4 September 2009
  (2009-09-04), XP055017152, Retrieved from the
  Internet:
  URL:http://www.xeptagen.net/download/produ
  cts/prostateic.datasheet.eng.pdf [retrieved on
  2012-01-23]**
- **Galosi: "Accuratezza diagnostica
  dell'immunocomplesso PSA-IgM nella diagnosi
  precoce del carcinoma prostatico", , 1 January
  2010 (2010-01-01), XP055017157, Retrieved from
  the Internet:
  URL:http://www.siuro.it/print/progetto/acc
  uratezza-diagnostica-dell%E2%80%99immunoc
  o mplesso-psa-igm-nella-diagnosi-precoce-del
  -carcinoma-p [retrieved on 2012-01-23]**
- **Xeptagen: "PROSTATE-IC PRODUCT
  PROFILE", , 24 September 2009 (2009-09-24),
  XP055017273, Retrieved from the Internet:
  URL:http://www.xeptagen.net/download/produ
  cts/prostateic.productprofile.eng.pdf [retrieved
  on 2012-01-23]**
- **Giorgio Fassina: "Applications and Case Studies
  of CC/CT/MD of CC/CT/MD in Diagnostics and
  Biotechnology", , 12 June 2009 (2009-06-12),
  XP055017261, Retrieved from the Internet:
  URL:www.ics.trieste.it/media/140298/df6336 .pd
  f [retrieved on 2012-01-23]**

**(Cont. next page)**

- **RIGOLLET ET AL: "Precurseurs et derives du PSA: application en clinique, revue de la litterature", IMMUNO ANALYSE ET BIOLOGIE SPECIALISE, ED. SCIENTIFIQUES ELSEVIER, PARIS, FR, vol. 22, no. 1, 8 March 2007 (2007-03-08), pages 1-4, XP005928941, ISSN: 0923-2532, DOI: 10.1016/J.IMMBIO.2006.11.001**
- **Siu: "LXXXIV Congresso Nazionale, Programma Congressuale", , 29 July 2011 (2011-07-29), XP055017308, Retrieved from the Internet: URL:http://www.siu.it/app/webroot/files/up loads/congressi%20SIU/SIU_programmma_29_ 07 .pdf [retrieved on 2012-01-24]**
- **ZANI D ET AL: "[Immunity and cancer: the role of PSA IgM Immune complexes for prostate cancer]", UROLOGIA : RIVISTA INTERNAZIONALE DI CULTURA UROLOGICA ; ORGANO UFFICIALE, WICHTIG, ITALY, vol. 77, no. 1, 25 March 2010 (2010-03-25) , pages 1-3, XP008147732, ISSN: 0391-5603 [retrieved on 2010-03-24]**
- **ANDREA GALLOTTA ET AL: "Clinical validation of the iXip index in avoiding unnecessary prostate biopsy: Results from a prospective multicenter study involving 426 patients", CANCER TREATMENT AND RESEARCH COMMUNICATIONS, vol. 10, 27 February 2017 (2017-02-27), pages 40-45, XP055464143, ISSN: 2468-2942, DOI: 10.1016/j.ctarc.2017.01.002**

## Description

This patent describes a method to diagnose the presence of prostate cancer. The method is able to detect the presence of prostate cancer in a population of men. On the basis of the present invention the method includes the steps of

a. Determination by enzyme-linked immunosorbent assay of the quantity of PSA-IgM occurring in a biological sample of the subject
b. Determination of prostate volume of the subject;
c. Definition of the age of the subject;
d. Conditional statement for patients with PSA-IgM/PV > 40 AU/ml2, corresponding to a predictive probability for prostate cancer set to 90%, wherein patients having values greater than 40 AU/ml2 are excluded from the method;
e. Normalization of PSA-IgM with age and prostate volume;
f. Determination of at least one of said clinical parameters of the subject that are not significantly correlated with PSA-IgM, as free PSA, PCA3, all precursors of the PSA, BPSA, and so on;
g. Mathematical combination of said value of PSA-IgM with one or more of said clinical parameters of the subject, defining an index or predictive value;
h. Correlation between this index or predictive value and a predetermined and established value with samples from patients with diagnosis of prostate cancer or known benign disease;
i. Determination of the risk of prostate cancer based on said correlation. and wherein that PSA-IgM is normalized (PSA-IgMnorm) with age and prostate volume as claimed in claim 1 and wherein said mathematical combination determines a predictive probability value for prostate cancer according to an index as claimed in claim 1.

## Background

The major cause of cancer death in men, second only to lung cancer, is prostate cancer (PCa); a man in nine over the age of 65 years is suffering from prostate cancer (Abate-Shen and Shen, Genes Dev, 2000, 14:2410; Ruijter et al., Endocr Rev, 1999, 20:22).

The technique most commonly used to verify the presence of prostate cancer are, respectively, the digital rectal examination (DRE) and the prostate specific antigen (PSA) test, which is considered a marker for prostate cancer because secreted only by prostate cells. A high serum level of PSA may indicate the presence of PCa. A healthy prostate, usually, produces a stable quantity of PSA and generally less than 4 nanograms per milliliter, whereas the tumor cells produce increasing quantities of PSA that should correspond to the severity of the cancer. A PSA level between 4 and 10 ng/ml may raise suspicion that the patient has prostate cancer, while levels above 50 ng/ml suggest, with greater certainty, the presence of advanced PCa.

The biopsy taking many samples in different regions of the prostate is the reference method for the formulation of a diagnosis of prostate cancer.

The type of treatment depends on the stage of cancer. Men with a life expectancy below 10 years, with a low Gleason score and a locally confined prostate cancer, often do not receive any treatment. The treatments for more aggressive tumors include those surgical as radical prostatectomy (RP), in which the prostate is completely removed (with or without nerve-sparing technique) or the radiation treatment, by beam radiation directed to the prostate (radiotherapy) or by low energy radioactive ions implanted in the prostate to kill the tumor cells at the local level (brachytherapy). The anti-androgen hormone therapy is used alone or, preferably, in combination with surgery or radiotherapy. Hormone therapy uses LH-RH (luteinizing hormone-releasing hormones) analogues, blocking testosterone production by the pituitary. Once the therapy is initiated, patients are subjected to injections of LH-RH analogues for the rest of their lives.

While surgical and hormonal treatments are often effective for localized prostate cancer, the advanced stage of the disease remains essentially incurable. The inhibition of androgens production is the most common therapy for advanced PCa, leading to massive apoptosis of androgen-dependent malignant cells and temporary tumor regression. In most cases, however, the tumor reemerges and can proliferate independently of androgen signals (hormone-resistant tumor).

The advent of prostate specific antigen screening has led to an increase in the diagnosis of PCa and has significantly reduced the deaths associated with this type of cancer. However, in only 20-30% of individuals undergoing biopsy cancer is found. (Kantoff and Talcott, Hematol. Oncol. Clinics N Amer, 1994, 8(3): 555)

In fact, an important limitation of the test of serum PSA is the low level of specificity to prostate cancer, especially in the intermediate range of detection of the PSA (4-10 ng/ml). In addition, elevated serum levels of PSA are often found in patients with benign diseases such as benign prostatic hyperplasia (BPH) and prostatitis, providing dubious information about the possible presence of cancer.

To increase the positive predictive value of the PSA assay alternative strategies have been developed that include the normalization with age, the determination of ratio of free to total PSA, the correlation with the prostate volume (PSA density) and the PSA levels over time (PSA velocity).

In any case, the sensitivity in the diagnosis of PCa remains poor, and the large-scale usage of PSA as first tool for the detection of cancer has dramatically increased the number of prostate biopsies performed (Jacobsen et al., JAMA, 1995, 274:1445), with an unavoidable increase of bioptic samples with negative results.

It is clear, therefore, that it is essential to identify new biomarkers and/or additional diagnostic parameters for the diagnosis of prostate cancer.

The patent depositories have previously identified a novel biomarker for prostate cancer, PSA-IgM, or the PSA immunocomplexed with IgM (European Application No. EP 10182565 and divisional application PCT / IT04/583, Beneduce et al. Prevent Cancer Detect., 2007, 31: 402-407; Verna et al.; Advanced Cancer Research 30; Abstract 196, 17 June 2010).

This new biomarker has a specificity for the detection of prostate cancer higher than PSA.

It has now surprisingly been found that the specificity of detection of prostate cancer with PSA-IgM can be further increased by correlating the values of PSA-IgM with the values of free PSA and total PSA, and other physiological parameters, such as the age of the patient and the prostate volume.

Is therefore a first object of the present invention a method for the diagnosis of prostate cancer, through, but not exclusively, the combination of circulating markers and physiological parameters.

The diagnosis of prostate cancer is the ability to distinguish patients with prostate cancer from those with benign diseases. The benign diseases include benign prostatic hyperplasia, prostatitis and other syndromes related to benign prostate.

The method comprises the following steps:

   a. Determination of the quantity of PSA-IgM occurring in a biological sample of the subject;
   b. Determination of the quantity of total PSA occurring in a biological sample of the subject;
   c. Determination of the quantity of free PSA occurring in a biological sample of the subject;
   d. Determination of prostate volume of the subject;
   e. Definition of the age of the subject.
   f. Correlation of the presence or absence of prostate cancer in the subject under examination with a value predetermined and established with patients samples diagnosed with prostate cancer or benign disease known on the basis of the mathematical combination of all or some of the quantities listed in points a. b. c. d. e. According to the present invention, the biological sample could be any physiological fluid, including, for example, blood, serum, plasma, seminal liquid and urine.

For the determination of different forms of PSA, including those complexed with IgM, are preferably used, but not exclusively, immunometric assays.

The prostate volume of the subject is determined, preferably but not exclusively, through the transrectal ultrasound examination or the estimated value of DRE. For example, the estimation of prostate volume expressed in ml could be determined considering the shape of the prostate as an ellipsoid as follows: 0.523 x width (cm) x height (cm) x length (cm). The width and height are measured in the axial plane while the length in the sagittal plane at the point of maximum diameter.

According to the application of the present invention, the acceptable range of total PSA is between 0 and 100 ng/ml but preferably higher than 4 ng/ml, or higher than 2 ng/ml for subjects with a family history of prostate cancer.

According to the application of the present invention, the range of acceptability of the prostate volume is between 10 and 300 ml.

According to the application of the present invention, PSA-IgM shall mean any forms of PSA complexed with immunoglobulins of class M (IgM).

According to the application of the present invention, the procedure of correlation could involve only the quantity of PSA-IgM, or a mathematical combination between PSA-IgM with one or more of the following quantities: total PSA, free PSA, prostate volume, age of the subject, PCA3, precursor forms and/or inactive PSA, but not only. The mathematical combination may perform through various functions such as addition, subtraction, division, multiplication, logarithm, exponential, inclusion/exclusion based on default values or combination of these.

Is therefore a second object of the present invention, the mathematical combination which can be divided into the following 4 steps:

   **Step 1 - conditional statements:** for patients with PSA-IgM/PV > 40 the predictive probability for prostate cancer (illustrated at the step 4) is set to 90%

   **Step 2 - normalization:** PSA-IgM was normalized (PSA-IgM$_{norm}$) with age and prostate volume as:

$$PSAIgM_{norm} = \frac{1}{(\beta_{21} \cdot PSAIgM)^{\beta_{22}}} \cdot \left( \frac{1}{(\beta_{31} \cdot PV)^{\beta_{32}}} - \frac{1}{Ln(\beta_{41} \cdot PV)^{\beta_{42}}} \right) \cdot (\beta_{11} \cdot Age)^{\beta_{12}}$$

**Spet 3 - logit:** fit a logistic regression model combining PSA-IgM$_{norm}$ with PSA in order to obtain an Immune complex predictive Index for prostate cancer (iXi):

$$iXi = \beta_0 + \beta_1 \cdot PSAIgM_{norm} + (\beta_{21} \cdot (tPSA + 0.1))^{\beta_{22}}$$

**Step 4 - probability:** finally, the predictive probability for prostate cancer (iXip) could be represented as follows:

$$iXip = \frac{1}{1 + e^{-iXi}}$$

This method, although not excluding other forms of association and/or correlation, allows to combine mathematically PSA-IgM with total PSA, prostate volume and age in order to generate an index and a predictive probability for prostate cancer, called iXip, which a range between 0% and 100%, calculated for each patient and it may be used to diagnose the presence of prostate cancer. Therefore, in function of the determined iXip index, patients can be divided by percentage of cancer risk according to Table 1.

Table 1: Correlations between prostate cancer risk and iXip index .

| Risk | iXip |
|---|---|
| Very Low | <20.0% |
| Low | 20.0% - 30.0% |
| Medium | 30.0% - 50.0% |
| High | 50.0% - 80.0% |
| Very High | >80% |

Is therefore a third object of the present invention the exclusion of patients from the biopsy procedure when iXip is lower than 20%.

Is therefore a fourth object of the present invention the certainty of positive biopsy for the presence of prostate cancer when iXip is higher than 80%.

**Examples**

**Example 1:** Analysis of serum of patients with prostate cancer or benign conditions and development of a patient profile.

*Materials and methods*

Male patients with clinical suspect of prostate cancer underwent transrectal ultrasound examination (TRUS) guided by a standardized sampling scheme.

Population criteria

Inclusion criteria were the following: high levels of total PSA (> 4.00 ng/ml or > 2.5 ng/ml for familiarity), high kinetics of PSA (> 0.75 ng/ml/year), suspicious digital rectal examinations, doubt TRUS exam for neoplasia. The exclusion criteria were: concomitant neoplasias, autoimmune diseases, active infections, steroid therapy, immunosuppressive therapy.

Determination of diagnostic parameters

Digital rectal examinations were deemed positive for PCa if there was nodularity or induration of the prostate or if the examiner judged the prostate to be suspicious for cancer based on other criteria, including asymmetry. (Friedman et al., Lancet, 1991, 337 (8756) :1526-9).

Prostate volume (PV) of each patient was assessed by TRUS examination. The estimated volume was calculated considering the prostate as an ellipsoid and the volume (ml) is determined by the following formula: $0.523 \times$ width (cm) x height (cm) x length (cm) (Terris et al., J Urol., 1991, 145:984-7). Width and height were measured on axial planes and craniocaudal length on sagittal planes at their greatest diameter. For all patients, serum samples were collected just before biopsy.

Serum PSA-IgM concentration was measured using Prostate-IC kit (XG007 - Xeptagen SpA). Concentrations were represented according to an arbitrary scale defined in the kit (AU/ml).

The concentrations of total and free PSA, represented in ng/ml, were determined by chemiluminescent immunometric assays (Diagnostic Products Corporation, Los Angeles, CA, USA).

*Statistical Analysis*

Statistical analyzes were performed using the software R version 2.12.0 (R Foundation for statistical computing). For all statistical comparisons, a p-value < 0.05 was accepted as statistically significant.

The correlation between variables such as tumor markers and diagnostic parameters has been defined according to the method of Pearson.

Levels of tumor markers and diagnostic parameters between groups of patients with PCa and benign conditions were compared using the Mann-Whitney test.

For each marker the ROC curve was constructed (receiver operating characteristic) that allowed to calculate the AUC (area under the ROC curve) (Bewick et al., Crit Care, 2005, 9 (1) :112-8).

AUCs were allowed to compare two markers (DeLong et al., Biometrics, 1988, 44 (3) :837-45).

*Results*

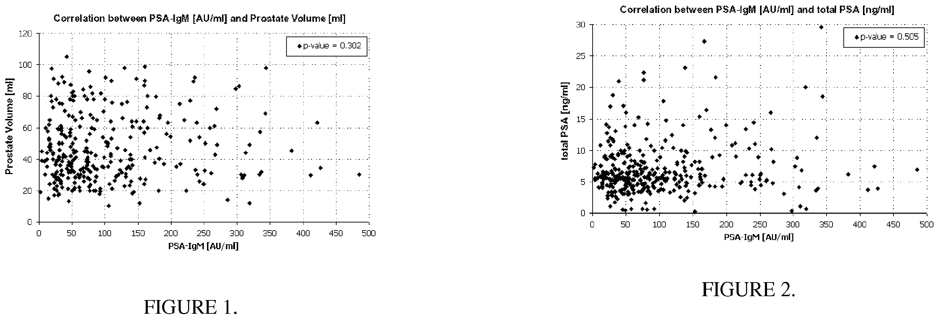

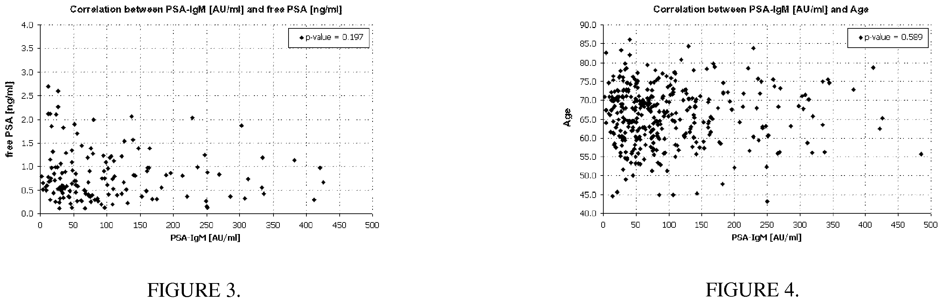

The lack of linear correlation between PSA-IgM and total PSA, free PSA, prostate volume and age is showed in Figures 1, 2, 3 and 4. In addition, Table 2 reports the p-value establishing no marker or diagnostic parameter are significantly correlated with PSA-IgM (p-value > 0.05). This demonstrates that the set of PSA-IgM, total PSA, free PSA, prostate volume and age represents a unique patient profile and the combined use of these parameters may allow a more accurate diagnosis for prostate cancer than the methods currently used in clinical practice.

The ROC curves and the analysis were carried out on approximately 400 serum samples with the diagnosis of cancer or benign conditions, with total PSA higher than 4 ng/ml or 2.5 ng/ml for familiarity, in which has also been determined the concentration of free PSA and PSA-IgM.

For each patient were also measured following diagnostic parameters: prostate volume and DRE.

The values of each marker and diagnostic parameter have been included in the R program in order to generate an analysis of the ROC curve.

The area under the ROC curve is the most widely used method for assessing the performance (in terms of diagnostic accuracy) of a marker as a predictor of cancer. Higher is the value of AUC, better is the diagnostic accuracy of the marker. The AUC ranges from 0.5 (no discrimination between patients and control subjects) to 1.0 (perfect test with a predictive value 100%).

Usually, the objective of AUC analysis is to maximize the capability to discriminate patients with cancer in order to reduce the biopsies to those who do not have cancer (false positives). Analyzing biological samples, for example, with serum total PSA to predict cancer, there is a balance between detection of true positives and false positives. For example, depending on the desired result, it may be preferable to detect 95% of tumors, but, due to the poor diagnostic accuracy of markers currently used in clinical practice, this range is inevitably accompanied by a high false positive rate for patients with benign disease.

The following examples are intended to demonstrate the capability and efficacy of the PSA-IgM in combination with total PSA, free PSA, prostate volume and age of the patient in a given set of criteria, but uses and cut-offs for these parameters are not limited to these examples. In addition, the representation of the patient profile and their combination with the PSA-IgM could be integrated with all the markers and/or diagnostic parameters that do not correlate significantly such as, for example, PCA3, all PSA precursors and BPSA, but not only.

Table 2: Correlation between PSA-IgM and total PSA, free PSA, prostate volume and age in 400 patients with prostate cancer and benign disease.

| Marker | Marker | R Pearson | p-value |
|---|---|---|---|
| PSA-IgM | Total PSA | 0.0311 | 0.505 |
| PSA-IgM | Free PSA | 0.0942 | 0.197 |
| PSA-IgM | Prostate Volume | 0.0510 | 0.302 |
| PSA-IgM | Age | 0.0250 | 0.589 |

**Example 2:** Serum analysis of men with prostate cancer and benign disease Following the criteria described in example 1, PSA-IgM, total PSA, prostate volume and age parameters were determined in a cohort of 197 patients.

The AUC of total PSA was 0.561 and does not allow, in any point of the curve, to discriminate patients with cancer from those with benign disease.

The combination of PSA-IgM, total PSA, prostate volume and age, generated an AUC of 0.731 which is high enough to discriminate between patients with cancer and with benign disease.

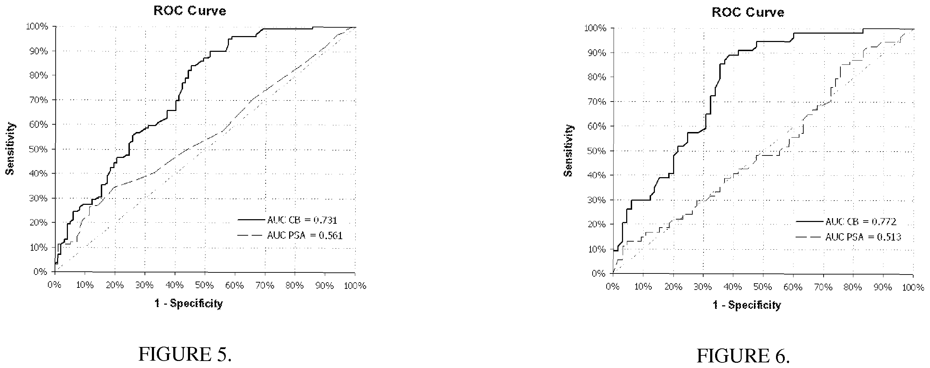

Figure 5 shows that the ROC curve of the combination is always significantly over the curve of total PSA; in addition, by suitably selecting cut-offs of the combination of PSA-IgM with the other parameters, it is possible to identify a number of patients with benign disease without excluding any patients with prostate cancer.

This example demonstrates that the combination of PSA-IgM with total PSA, prostate volume and age could identify patients without cancer and its use in the clinical practice, but is not limited to this sole application, may reduce the number of biopsies with negative results by 7.1% (iXip < 20%) without excluding any positive biopsy or by 19.8% (iXip < 30%) with a loss of positive biopsies of 2.0%.

**Example 3:** Serum analysis of men with prostate cancer and benign disease with total PSA concentration between 4 and 10 ng/ml

Following the criteria described in example 1, PSA-IgM, total PSA, prostate volume and age parameters were determined in a cohort of 120 patients.

The AUC of total PSA was 0.513 and does not allow, in any point of the curve, to discriminate patients with cancer from those with benign disease.

The combination of PSA-IgM, total PSA, prostate volume and age, generated an AUC of 0.772 which is high enough to discriminate between patients with cancer and with benign disease.

Figure 6 shows that the ROC curve of the combination is always significantly over the curve of total PSA; in addition, by suitably selecting cut-offs of the combination of PSA-IgM with the other parameters, it is possible to identify a number of patients with benign disease without excluding any patients with prostate cancer.

A major limitation of the serum total PSA test is the lack of sensitivity and specificity to prostate cancer especially in the range between 4 and 10 ng/ml.

This example demonstrates that the combination of PSA-IgM with total PSA, prostate volume and age could identify patients without cancer with PSA levels between 4 and 10 ng/ml and its use in the clinical practice, but is not limited to this sole application, may reduce the number of biopsies with negative results by 9.2% without excluding any positive biopsy or by 21.7% with a loss of positive biopsies of 0.8%.

**Example 4:** Serum analysis of men with prostate cancer and benign disease with total PSA concentration greater than 10 ng/ml

Following the criteria described in example 1, PSA-IgM, total PSA, prostate volume and age parameters were determined in a cohort of 42 patients.

The AUC of total PSA was 0.632 and does not allow, in any point of the curve, to discriminate patients with cancer from those with benign disease.

The combination of PSA-IgM, total PSA, prostate volume and age, generated an AUC of 0.810 which is high enough to discriminate between patients with cancer and with benign disease.

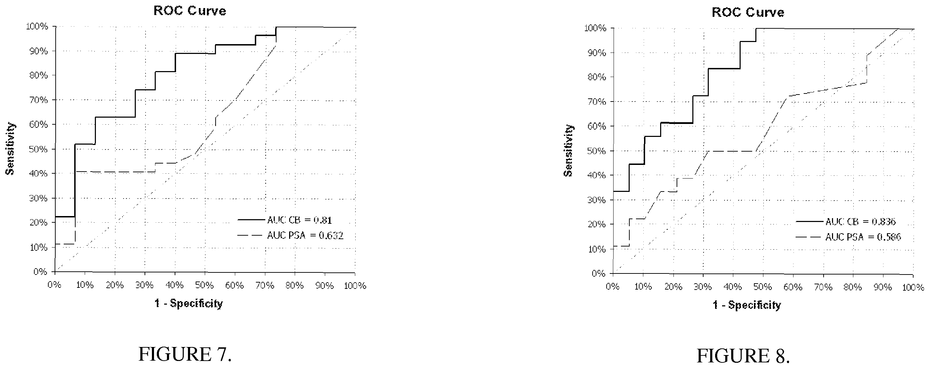

Figure 7 shows that the ROC curve of the combination is always significantly over the curve of total PSA; in addition, by suitably selecting cut-offs of the combination of PSA-IgM with the other parameters, it is possible to identify a number of patients with benign disease without excluding any patients with prostate cancer.

This example demonstrates that the combination of PSA-IgM with total PSA, prostate volume and age could identify patients without cancer and its use in clinical practice, but is not limited to this sole application, may reduce the number of biopsies with negative results by 9.5% without excluding any positive biopsy.

**Example 5:** Serum analysis of men under 60 years with prostate cancer and benign disease

Following the criteria described in example 1, PSA-IgM, total PSA, prostate volume and age parameters were determined in a cohort of 37 patients.

The AUC of total PSA was 0.586 and does not allow, in any point of the curve, to discriminate patients with cancer from those with benign disease.

The combination of PSA-IgM, total PSA, prostate volume and age, generated an AUC of 0.836 which is high enough to discriminate between patients with cancer and with benign disease.

Figure 8 shows that the ROC curve of the combination is always significantly over the curve of total PSA; in addition, by suitably selecting cut-offs of the combination of PSA-IgM with the other parameters, it is possible to identify a number of patients with benign disease without excluding any patients with prostate cancer.

A very important application in the diagnosis of prostate cancer is to identify this pathology in patients with a life expectancy greater than 10 years in which the tumor is more aggressive and, therefore, generally in subjects under 60 years .

This example demonstrates that the combination of PSA-IgM with total PSA, prostate volume and age could identify patients without cancer with PSA levels between 4 and 10 ng/ml and its use in clinical practice, but is not limited to this single application, may reduce the number of biopsies with negative results by 27.0% without excluding any positive biopsy.

**Example 6:** Serum analysis of men with prostate cancer and benign disease with digital rectal examination suspicious for cancer

Following the criteria described in example 1, PSA-IgM, total PSA, prostate volume and age parameters were determined in a cohort of 75 patients.

The AUC of total PSA was 0.645 and does not allow, in any point of the curve, to discriminate patients with cancer from those with benign disease.

The combination of PSA-IgM, total PSA, prostate volume and age, generated an AUC of 0.753 which is high enough to discriminate between patients with cancer and with benign disease.

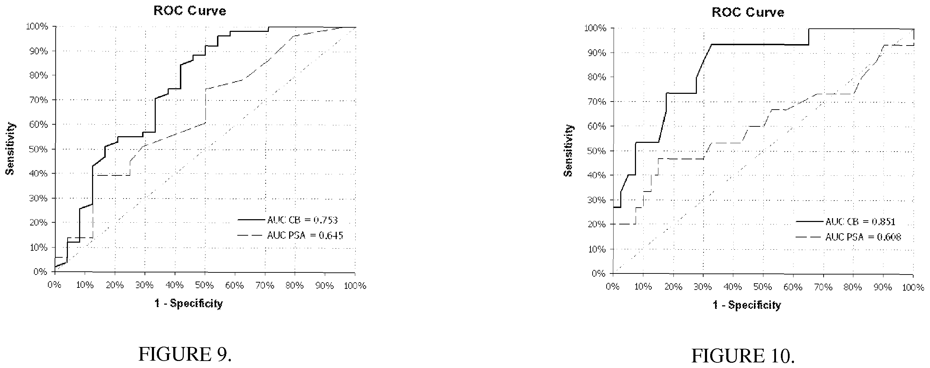

Figure 9 shows that the ROC curve of the combination is always significantly over the curve of total PSA; in addition, by suitably selecting cut-offs of the combination of PSA-IgM with the other parameters, it is possible to identify a number of patients with benign disease without excluding any patient with prostate cancer.

In the current clinical practice all patients with positive digital rectal examination and PSA greater than 4 ng/ml undergo biopsy.

This example demonstrates that the combination of PSA-IgM with total PSA, prostate volume and age could identify patients without cancer with PSA levels between 4 and 10 ng/ml and its use in clinical practice, but is not limited to this single application, may reduce the number of biopsies with negative results by 9.3% without excluding any positive biopsy or by 13.3% with a loss of positive biopsies of 1.3%.

**Example 7:** Serum analysis of men with prostate cancer and benign disease with prostate volume greater than 60 ml.

Following the criteria described in example 1, PSA-IgM, total PSA, prostate volume and age parameters were determined in a cohort of 55 patients.

The AUC of total PSA was 0.608 and does not allow, in any point of the curve, to discriminate patients with cancer from those with benign disease.

The combination of PSA-IgM, total PSA, prostate volume and age, generated an AUC of 0.851 which is high enough to discriminate between patients with cancer and with benign disease.

Figure 10 shows that the ROC curve of the combination is always significantly over the curve of total PSA; in addition, by suitably selecting cut-offs of the combination of PSA-IgM with the other parameters, it is possible to identify a number of patients with benign disease without excluding any patient with prostate cancer.

Published studies have shown that benign conditions such as benign prostatic hypertrophy cause an increase of prostate volume and, in addition, it is clear that considerable prostate volumes can give rise to doubts at the diagnosis of cancer.

This example demonstrates that the combination of PSA-IgM with total PSA, prostate volume and age could identify patients without cancer with PSA levels between 4 and 10 ng/ml and its use in clinical practice, but is not limited to this single application, may reduce the number of biopsies with negative results by 25.5% without excluding any positive biopsy.

**Example 8:** Serum analysis of men with prostate cancer and benign disease

Following the criteria described in example 1, PSA-IgM, total PSA, prostate volume and age parameters were determined in a cohort of 151 patients.

The AUC of total PSA was 0.557 and does not allow, in any point of the curve, to discriminate patients with cancer from those with benign disease.

The combination of PSA-IgM, total PSA, prostate volume and age, generated an AUC of 0.752 which is high enough to discriminate between patients with cancer and with benign disease.

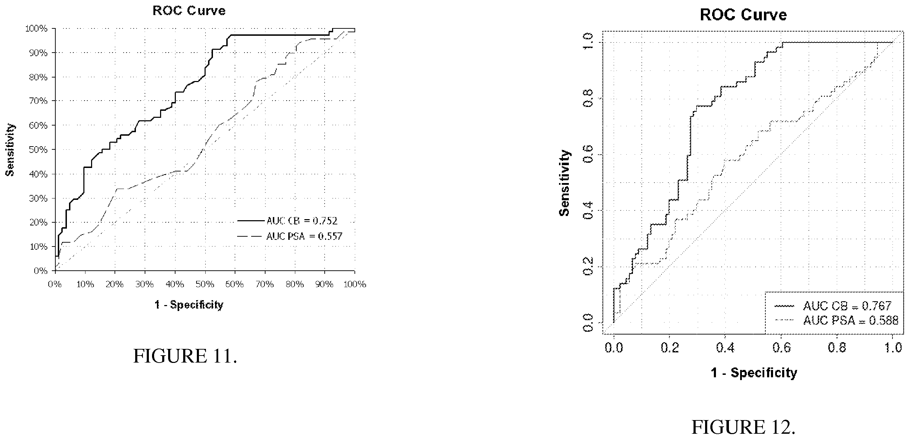

Figure 11 shows that the ROC curve of the combination is always significantly over the curve of total PSA; in addition, by suitably selecting cut-offs of the combination of PSA-IgM with the other parameters, it is possible to identify a number of patients with benign disease without excluding any patient with prostate cancer.

This example shows an alternative combination with respect to example 3, which also allows to identify patients without cancer and its use in clinical practice, but is not limited to this sole application, may reduce the number of biopsies with negative results by 18.5% with a loss of positive biopsies of 1.3%.

**Example 9:** Serum analysis of men with prostate cancer and benign disease Following the criteria described in example 1, PSA-IgM, prostate volume and phi parameters were determined in a cohort of 148 patients.

The AUC of total PSA was 0.588 and does not allow discriminating patients with cancer from those with benign disease.

The combination of PSA-IgM, prostate volume and phi generated an AUC of 0.767 which is high enough to discriminate between patients with cancer and with benign disease.

Figure 12 shows that the ROC curve of the combination is significantly over the curve of total PSA; in addition, by suitably selecting cut-offs of the combination of PSA-IgM with the other parameters, it is possible to identify a number of patients with benign disease without excluding any patient with prostate cancer.

This example demonstrates that the combination of PSA-IgM with prostate volume and phi could identify patients without cancer and its use in clinical practice, but is not limited to this sole application, may reduce the number of biopsies with negative results by 39.6% without excluding any positive biopsy.

**Example 10:** Serum analysis of men with prostate cancer and benign disease Following the criteria described in example 1, PSA-IgM, prostate volume, age, total PSA and p2PSA parameters were determined in a cohort of 148 patients.

The AUC of total PSA was 0.588 and does not allow discriminating patients with cancer from those with benign disease.

The combination of PSA-IgM, prostate volume, age, total PSA and p2PSA generated an AUC of 0.782 which is high enough to discriminate between patients with cancer and with benign disease.

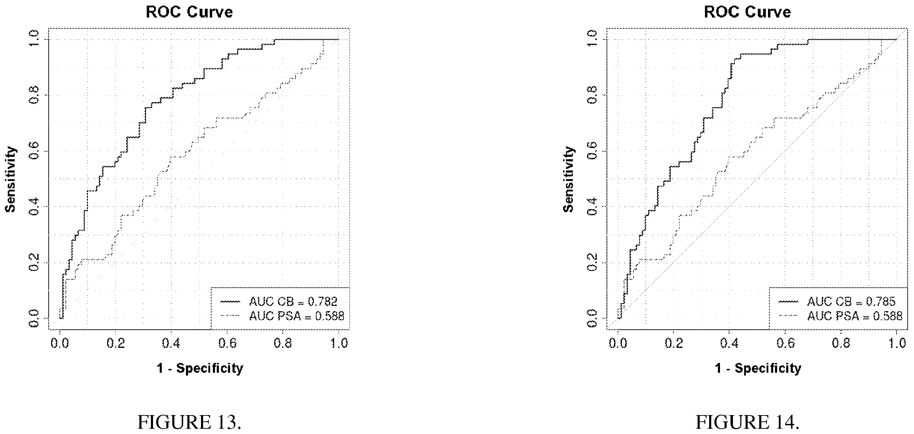

Figure 13 shows that the ROC curve of the combination is significantly over the curve of total PSA; in addition, by suitably selecting cut-offs of the combination of PSA-IgM with the other parameters, it is possible to identify a number of patients with benign disease without excluding any patient with prostate cancer.

This example demonstrates that the combination of PSA-IgM with prostate volume, age, total PSA and p2PSA could identify patients without cancer and its use in clinical practice, but is not limited to this sole application, may reduce the number of biopsies with negative results by 23.1% without excluding any positive biopsy. **Example 11:** Serum analysis of men with prostate cancer and benign disease Following the criteria described in example 1, PSA-IgM, prostate volume, age, total PSA, phi and PCA3 parameters were determined in a cohort of 148 patients.

The AUC of total PSA was 0.588 and does not allow discriminating patients with cancer from those with benign disease.

The combination of PSA-IgM, prostate volume, age, total PSA, phi and PCA3 generated an AUC of 0.785 which is high enough to discriminate between patients with cancer and with benign disease.

Figure 14 shows that the ROC curve of the combination is significantly over the curve of total PSA; in addition, by suitably selecting cut-offs of the combination of PSA-IgM with the other parameters, it is possible to identify a number of patients with benign disease without excluding any patient with prostate cancer.

This example demonstrates that the combination of PSA-IgM with prostate volume, age, total PSA, phi and PCA3 could identify patients without cancer and its use in clinical practice, but is not limited to this sole application, may reduce the number of biopsies with negative results by 31.9% without excluding any positive biopsy.

## Description of Figures

    **Figure 1:** Correlation between PSA-IgM [AU/ml] and Prostate Volume [ml] in 400 patients with prostate cancer and benign disease.

    **Figure 2:** Correlation between PSA-IgM [AU/ml] and total PSA [ng/mL] in 400 patients with prostate cancer and benign disease.

    **Figure 3:** Correlation between PSA-IgM [AU/ml] and free PSA [ng/mL] in 400 patients with prostate cancer and benign disease.

    **Figure 4:** Correlation between PSA-IgM [AU/ml] and Age in 400 patients with prostate cancer and benign disease.

    **Figure 5:** ROC curve of 197 patients with prostate cancer and benign conditions of a possible combination of PSA-IgM, total PSA, prostate volume and age (CB) and total PSA.

    **Figure 6:** ROC curve of 120 patients with prostate cancer and benign conditions of a possible combination of PSA-IgM, total PSA, prostate volume and age (CB) and total PSA.

**Figure 7:** ROC curve of 42 patients with prostate cancer and benign conditions of a possible combination of PSA-IgM, total PSA, prostate volume and age (CB) and total PSA.

**Figure 8:** ROC curve of 37 patients with prostate cancer and benign conditions of a possible combination of PSA-IgM, total PSA, prostate volume and age (CB) and total PSA.

**Figure 9:** ROC curve of 75 patients with prostate cancer and benign conditions of a possible combination of PSA-IgM, total PSA, prostate volume and age (CB) and total PSA.

**Figure 10:** ROC curve of 55 patients with prostate cancer and benign conditions of a possible combination of PSA-IgM, total PSA, prostate volume and age (CB) and total PSA.

**Figure 11:** ROC curve of 151 patients with prostate cancer and benign conditions of a possible combination of IgM-PSA, free PSA, prostate volume and age (CB) and total PSA.

**Figure 12:** ROC curve of 148 patients with prostate cancer and benign conditions of a possible combination of PSA-IgM, phi and prostate volume (CB) and total PSA.

**Figure 13:** ROC curve of 148 patients with prostate cancer and benign conditions of a possible combination of PSA-IgM, prostate volume, age, total PSA and p2PSA (CB) and total PSA.

**Figure 14:** ROC curve of 148 patients with prostate cancer and benign conditions of a possible combination of PSA-IgM, prostate volume, age, total PSA, phi and PCA3 (CB) and total PSA.

## Discussion

These examples demonstrate that the combination of PSA-IgM with total PSA, free PSA, age and prostate volume allows increasing the diagnostic accuracy for PCa compared to total PSA.

We have previously shown that the PSA-IgM allows to more accurately detecting the presence of cancer in populations of patients at risk compared to total PSA (European Application No. EP 10182565 and divisional application PCT / IT04/583, Beneduce et al. Prevent Cancer Detect., 2007, 31: 402-407), which is used as a reference marker in patients at risk.

In order to improve the capability of prostate cancer identification it has been surprisingly discovered that the combination of PSA-IgM with not significantly correlated markers, which therefore allows to define a unique profile of the patient, provides a significant increase in diagnosis of PCa thus providing a better performance in discriminating cancer patients from those with other benign conditions.

This new and surprising discovery suggests a complex relationship between PSA-IgM and many markers and diagnostic parameters of the patient under consideration, in addition, the diagnostic accuracy of the combined marker can be applied to any value of total PSA ensuring a wide range of clinical applications.

The examples given are only intended to demonstrate the applicability of the combination of PSA-IgM with other markers provided that they are not significantly correlated, to detect prostate cancer, and to suggest some relevant examples of application, but these examples are not intended to limit other potential applications. Therefore, with reference to the above description and the accompanying tables, the following claims are made.

## Claims

**1.** Method for determining a prostate cancer risk, for distinguishing prostate cancer from benign prostate disease in a subject whatever, comprising the combination of two or more clinical parameters of the subject to determine the risk of prostate cancer, where one of these clinical parameters is the quantity of PSA-IgM occurring in a biological sample of the subject, combined with at least one additional clinical parameter that is not significantly correlated with PSA-IgM, **characterized in that** said method comprises the following steps:

a. Determination by enzyme-linked immunosorbent assay of the quantity of PSA-IgM occurring in a biological sample of the subject

b. Determination of prostate volume of the subject;

c. Definition of the age of the subject;

d. Conditional statement for patients with PSA-IgM/PV > 40 AU/ml$^2$, corresponding to a predictive probability for prostate cancer set to 90%, wherein patients having values greater than 40 AU/ml$^2$ are excluded from the method;

e. Normalization of PSA-IgM with age and prostate volume;

f. Determination of at least one of said clinical parameters of the subject that are not significantly correlated with PSA-IgM, as free PSA, PCA3, all precursors of the PSA, BPSA, and so on;

g. Mathematical combination of said value of PSA-IgM with one or more of said clinical parameters of the subject, defining an index or predictive value;

h. Correlation between this index or predictive value and a predetermined and established value with samples from patients with diagnosis of prostate cancer or known benign disease;
**i.** Determination of the risk of prostate cancer based on said correlation.

and wherein that PSA-IgM is normalized (PSA-IgMnorm) with age and prostate volume as:

$$PSAIgM_{norm} = \frac{1}{(\beta_{21} \cdot PSAIgM)^{\beta_{22}}} \cdot \left( \frac{1}{(\beta_{31} \cdot PV)^{\beta_{32}}} - \frac{1}{Ln(\beta_{41} \cdot PV)^{\beta_{42}}} \right) \cdot (\beta_{11} \cdot Age)^{\beta_{12}}$$

and wherein said mathematical combination determines a predictive probability value for prostate cancer according to an index (iXip), wherein:

$$iXi = \beta_0 + \beta_1 \cdot PSAIgM_{norm} + (\beta_{21} \cdot (tPSA + 0.1))^{\beta_{22}}$$

and

$$iXip = \frac{1}{1 + e^{-iXi}} \, .$$

and wherein

| Risk | iXip |
|------|------|
| Very Low | <20.0% |
| Low | 20.0% - 30.0% |
| Medium | 30.0% - 50.0% |
| High | 50.0% - 80.0% |
| Very High | >80% |

2. Method, according to claim 1 where by using said index iXip is possible to identify subjects who will be negative at biopsy for the presence of prostate cancer and/or those subject who will be positive at biopsy for the presence of prostate cancer, wherein the correlation between prostate cancer risk and said index (iXip) is: very low risk with said index <20.0%; low risk with said index comprised between 20.0% and 30.0%; medium risk with said index comprised between 30.0% and 50.0%; high risk with said index comprised between 50.0% and 80.0%; very high risk with said index >80%.

3. Method, according to previous claims, where the mathematical combination is between PSA-IgM, total PSA, prostate volume and age.

4. Method, according to previous claims, where the mathematical combination is between PSA-IgM, free PSA, prostate volume and age.

5. Method, according to previous claims, where the mathematical combination is between PSA-IgM, free PSA, total PSA, prostate volume and age.

6. Method, according to previous claims, where the mathematical combination is between PSA-IgM and a precursors of the PSA.

7. Method, according to previous claims, where the mathematical combination is between PSA-IgM, a precursors of the PSA and prostate volume.

8. Method, according to previous claims, where the criteria to include the subject are:

- the value of total PSA is greater than 2.5 ng/ml; or
- the value of total PSA is greater than 4 ng/ml; or
- the value of total PSA is between 2.5 and 10 ng/ml; or
- the value of total PSA is between 4 and 10 ng/ml; or
- the value of total PSA is greater than 10 ng/ml; and/or
- the prostate volume is greater than 60 ml; and/or
- the age is less than 60 years; and/or
- digital rectal examination is positive for the presence of prostate cancer; and/or
- the ratio of free PSA to total PSA is between 1 and 50%; or
- the ratio of free PSA to total PSA is greater than 20%; or
- the ratio of free PSA to total PSA is greater than 25%.

## Patentansprüche

1. Verfahren zur Bestimmung eines Prostatakrebsrisikos, zur Unterscheidung zwischen Prostatakrebs und einer gutartigen Prostataerkrankung in jeglichem Proband, die Kombination von zwei oder mehreren klinischen Parametern des Probanden umfassend, um das Prostatakrebsrisiko zu bestimmen, wobei einer dieser klinischen Parameter die in einer biologischen Probe des Probanden enthaltene Menge an PSA-IgM ist, kombiniert mit wenigstens einem zusätzlichen klinischen Parameter, der nicht in signifikanter Weise mit PSA-IgM in Korrelation steht, **dadurch gekennzeichnet, dass** das besagte Verfahren folgende Schritte umfasst:

a. Bestimmung der in einer biologischen Probe des Probanden enthaltene Menge PSA-IgM durch enzymatische Immunadsorptionsanalyse;
b. Bestimmung des Prostatavolumens des Probanden;
c. Feststellung des Alters des Probanden;
d. Bedingungsfeststellung für Patienten mit PSA-IgM/PV > 40 AU/ml, entsprechend einer auf 90 % gesetzten prädiktiven Prostatakrebs-Wahrscheinlichkeit; wobei Patienten mit Werten über 40 AU/ml vom Verfahren ausgeschlossen werden;
e. Normalisierung von PSA-IgM bezüglich Alter und Prostatavolumen;
f. Bestimmung von wenigstens einem der besagten klinischen Parametern des Probanden, die nicht signifikant mit PSA-IgM in Korrelation stehen, wie freies PSA, PC A3, alle Vorstufen von PSA, BPSA, und so weiter;
g. Rechnerische Kombination des besagten Werts PSA-IgM mit einem oder mehreren der besagten klinischen Parameter des Probanden, unter Definition einer Kennzahl bzw. eines prädiktiven Werts;
h. Korrelation zwischen dieser Kennzahl bzw. diesem prädiktivem Wert und einem vorbestimmten und festgesetzten Wert mit Proben von Patienten mit diagnostiziertem Prostatakrebs oder einer bekannten gutartigen Erkrankung;
i. Bestimmung des Prostatakrebsrisikos auf Grundlage der besagten Korrelation;

und wobei dieser PSA-IgM-Wert bezüglich Alter und Prostatavolumen normalisiert ist (PSA-IgMnorm) als:

$$PSA\lg M_{norm} = \frac{1}{(\beta_{21} \cdot PSA\lg M)^{\beta_{22}}} \cdot \left( \frac{1}{(\beta_{31} \cdot PV)^{\beta_{32}}} - \frac{1}{Ln(\beta_{41} \cdot PV)^{\beta_{42}}} \right) \cdot (\beta_{11} \cdot Alter)^{\beta_{12}}$$

und wobei die besagte rechnerische Kombination einen prädiktiven Wahrscheinlichkeitswert für Prostatakrebs entsprechend einer Kennzahl (iXip) bestimmt, wobei:

$$iXi = \beta_0 + \beta_1 \cdot PSA\lg M_{norm} + (\beta_{21} \cdot (tPSA + 0.1))^{\beta_{22}}$$

und

$$iXip = \frac{1}{1 + e^{-iXi}}$$

und wobei

| Risiko | iKip |
|---|---|
| Sehr gering | <20,0 % |
| Gering | 20,0 % - 30,0 % |
| Mittel | 30,0 % - 50,0 % |
| Hoch | 50,0 % - 80,0 % |
| Sehr hoch | >80% |

**2.** Verfahren nach Patentanspruch 1, wobei es durch Anwendung der besagten Kennzahl iXip möglich ist, Probanden zu identifizieren, deren Biopsie zur Erkennung von Prostatakrebs negativ ausfallen wird und/oder jene Probanden, deren Biopsie zur Erkennung von Prostatakrebs positiv ausfallen wird, wobei die Korrelation zwischen Prostatakrebsrisiko und der besagten Kennzahl (iXip) wie folgt lautet: sehr geringes Risiko mit der besagten Kennzahl <20,0 %; geringes Risiko mit der besagten Kennzahl zwischen 20,0 % und 30,0 %; mittleres Risiko mit der besagten Kennzahl zwischen 30,0 % und 50,0 %; hohes Risiko mit der besagten Kennzahl zwischen 50,0 % und 80,0 %; sehr hohes Risiko mit der besagten Kennzahl >80 %.

**3.** Verfahren nach den vorstehenden Patentansprüchen, wobei die rechnerische Kombination aus PSA-IgM, Gesamtwert PSA, Prostatavolumen und Alter besteht.

**4.** Verfahren nach den vorstehenden Patentansprüchen, wobei die rechnerische Kombination aus PSA-IgM, freiem PSA, Prostatavolumen und Alter besteht.

**5.** Verfahren nach den vorstehenden Patentansprüchen, wobei die rechnerische Kombination aus PSA-IgM, freiem PSA, Gesamt-PSA, Prostatavolumen und Alter besteht.

**6.** Verfahren nach den vorstehenden Patentansprüchen, wobei die rechnerische Kombination aus PSA-IgM und einer Vorstufe des PSA besteht.

**7.** Verfahren nach den vorstehenden Patentansprüchen, wobei die rechnerische Kombination aus PSA-IgM, einer Vorstufe des PSA und dem Prostatavolumen besteht.

**8.** Verfahren nach den vorstehenden Patentansprüchen, wobei die Kriterien zur Einschließung des Probanden Folgende sind:

- das Gesamt-PSA ist größer als 2,5 ng/ml; oder
- das Gesamt-PSA ist größer als 4 ng/ml; oder
- das Gesamt-PSA liegt zwischen 2,5 und 10 ng/ml; oder
- das Gesamt-PSA liegt zwischen 4 und 10 ng/ml; oder
- das Gesamt-PSA ist größer als 10 ng/ml; und/oder
- das Prostatavolumen ist größer als 60 ml; und/oder
- das Alter liegt unter 60 Jahren; und/oder
- die digital-rektale Untersuchung auf Prostatakrebs resultiert positiv; und/oder
- das Verhältnis von freiem PSA zum Gesamt-PSA liegt zwischen 1 und 50 %; oder
- das Verhältnis von freiem PSA zum Gesamt-PSA ist größer als 20 %; oder
- das Verhältnis von freiem PSA zum Gesamt-PSA ist größer als 25 %.

**Revendications**

**1.** Méthode pour déterminer le risque de cancer de la prostate, pour distinguer le cancer de la prostate de la maladie bénigne de la prostate dans une personne quelconque, comprenant la combinaison de deux ou plusieurs paramètres cliniques de la personne concernée pour déterminer le risque de cancer de la prostate, où un desdits paramètres cliniques est la quantité de PSA-IgM présente dans un échantillon biologique de la personne en question, combinée avec au moins un paramètre clinique supplémentaire qui n'est pas considérablement corrélé avec le PSA-IgM, **caractérisée en ce que** ladite méthode comprend les phases suivantes :

    a. détermination de la quantité de PSA-IgM présente dans un échantillon biologique de la personne concernée par essai d'immuno-absorption enzymatique ;
    b. détermination du volume de la prostate de la personne concernée ;
    c. définition de l'âge de la personne concernée ;
    d. déclaration conditionnelle pour les patients avec PSA-IgM/PV > 40 AU/ml$^2$, correspondant à une probabilité préventive pour le cancer de la prostate fixée à 90 %, où les patients ayant des valeurs supérieures à 40 AU/ml$^2$ sont exclus de la méthode ;
    e. normalisation de PSA-IgM avec l'âge et le volume de la prostate ;
    f. détermination d'au moins un desdits paramètres cliniques de la personne concernée qui ne sont pas considérablement corrélés avec le PSA-IgM, comme PSA libre, PCA3, tous précurseurs du PSA, BPSA, etc. ;
    g. combinaison mathématique de ladite valeur de PSA-IgM avec un ou plusieurs desdits paramètres cliniques de la personne concernée, définissant un indice ou une valeur prévisionnelle ;
    h. corrélation entre ledit indice ou la valeur prévisionnelle et une valeur prédéterminée et établie avec des échantillons de patients ayant reçu un diagnostic de cancer de la prostate ou une maladie bénigne connue ;
    i. détermination du risque de cancer de la prostate basée sur ladite corrélation,

et où ledit PSA-IgM est normalisé (PSA-IgMnorm) avec l'âge et le volume de la prostate comme :

$$PSA\lg M_{norm} = \frac{1}{(\beta_{21} \cdot PSA\lg M)^{\beta_{22}}} \cdot \left( \frac{1}{(\beta_{31} \cdot PV)^{\beta_{32}}} - \frac{1}{Ln(\beta_{41} \cdot PV)^{\beta_{42}}} \right) \cdot (\beta_{11} \cdot \hat{A}ge)^{\beta_{12}}$$

et où ladite combinaison mathématique détermine une valeur de probabilité prévisionnelle pour le cancer de la prostate selon un indice (iXip), où :

$$iXi = \beta_0 + \beta_1 \cdot PSA\lg M_{norm} + (\beta_{21} \cdot (tPSA + 0.1))^{\beta_{22}}$$

et

$$iXip = \frac{1}{1 + e^{-iXi}}$$

et où

| Risque | iXip |
|---|---|
| Très bas | <20,0 % |
| Bas | 20,0 % - 30,0 % |
| Moyen | 30,0 % - 50,0 % |
| Élevé | 50,0 % - 80,0 % |
| Très élevé | >80,0 % |

**2.** Méthode, selon la revendication 1, où en utilisant ledit indice iXip il est possible d'identifier des personnes qui seront négatives à la biopsie pour la présence de cancer de la prostate et/ou lesdites personnes qui seront positives à la biopsie pour la présence de cancer de la prostate, où la corrélation entre le risque de cancer de la prostate et ledit indice (iXip) est : un risque très bas avec ledit indice <20,0 % ; un risque bas avec ledit indice compris entre 20,0 % et 30,0 % ; un risque moyen avec ledit indice compris entre 30,0 % et 50,0 % ; un risque élevé avec ledit indice compris entre 50,0 % et 80,0 % ; un risque très élevé avec ledit indice >80 %.

3. Méthode selon les revendications précédentes, où la combinaison mathématique est entre PSA-IgM, PSA total, volume de la prostate et âge.

4. Méthode selon les revendications précédentes, où la combinaison mathématique est entre PSA-IgM, PSA libre, volume de la prostate et âge.

5. Méthode selon les revendications précédentes, où la combinaison mathématique est entre PSA-IgM, PSA libre, PSA total, volume de la prostate et âge.

6. Méthode selon les revendications précédentes, où la combinaison mathématique est entre PSA-IgM et un précurseur du PSA.

7. Méthode selon les revendications précédentes, où la combinaison mathématique est entre PSA-IgM, un précurseur du PSA et le volume de la prostate.

8. Méthode selon les revendications précédentes, où les critères pour inclure la personne concernée sont :

   • la valeur de PSA total est supérieure à 2,5 ng/ml ; ou
   • la valeur de PSA total est supérieure à 4 ng/ml ; ou
   • la valeur de PSA total est comprise entre 2,5 et 10 ng/ml ; ou
   • la valeur de PSA total est comprise entre 4 et 10 ng/ml ; ou
   • la valeur de PSA total est supérieure à 10 ng/ml ; et/ou
   • le volume de la prostate est supérieur à 60 ml ; et/ou
   • l'âge est inférieur à 60 ans ; et/ou
   • l'examen rectal digital est positif pour la présence du cancer de la prostate ; et/ou
   • le rapport entre PSA libre et PSA total est compris entre 1 et 50 % ; ou
   • le rapport entre PSA libre et PSA total est supérieur à 20 % ; ou
   • le rapport entre PSA libre et PSA total est supérieur à 25 %.

Correlation between PSA-IgM [AU/ml] and Prostate Volume [ml]

FIGURE 1.

Correlation between PSA-IgM [AU/ml] and total PSA [ng/ml]

FIGURE 2.

FIGURE 3.

FIGURE 4.

EP 2 766 729 B1

FIGURE 6.

FIGURE 5.

FIGURE 8.

FIGURE 7.

FIGURE 10.

FIGURE 9.

FIGURE 12.

FIGURE 11.

FIGURE 14.

FIGURE 13.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 10182565 A

- IT 04583 W

**Non-patent literature cited in the description**

- **ABATE-SHEN ; SHEN.** *Genes Dev,* 2000, vol. 14, 2410
- **RUIJTER et al.** *Endocr Rev,* 1999, vol. 20, 22
- **KANTOFF ; TALCOTT.** *Hematol. Oncol. Clinics N Amer,* 1994, vol. 8 (3), 555
- **JACOBSEN et al.** *JAMA,* 1995, vol. 274, 1445
- **BENEDUCE et al.** *Prevent Cancer Detect.,* 2007, vol. 31, 402-407

- **VERNA et al.** *Advanced Cancer Research,* 17 June 2010, vol. 30, 196
- **FRIEDMAN et al.** *Lancet,* 1991, vol. 337 (8756), 1526-9
- **TERRIS et al.** *J Urol.,* 1991, vol. 145, 984-7
- **BEWICK et al.** *Crit Care,* 2005, vol. 9 (1), 112-8
- **DELONG et al.** *Biometrics,* 1988, vol. 44 (3), 837-45